# NOUVEAU FASCICULE DE BREVET EUROPEEN

(11) **EP 0 349 429 B2**
(45) Date de publication et mention de la décision concernant l'opposition: **20.11.1996**
(45) Mention de la délivrance du brevet: 24.02.1993
(21) Numéro de dépôt: 89401857.1
(22) Date de dépôt: 28.06.1989
(51) Int. Cl.: A61K 9/127

(54) **Procédé de préparation de systèmes colloidaux dispersibles de lipides amphiphiles sous forme de liposomes submicroniques**
Verfahren zur Herstellung dispergierbarer kolloidaler Systeme aus amphiphilen Lipiden in Form von Liposomen in Submikrongrösse
Process for the preparation of dispersible colloidal systems of amphiphilic lipids in the form of submicronic liposomes

(30) Priorité: 30.06.1988 FR 8808874
(43) Date de publication de la demande: 03.01.1990
(73) Titulaire: CENTRE NATIONAL DE LA RECHERCHE SCIENTIFIQUE (CNRS), 75700 Paris Cedex 07 (FR)
(72) Inventeur: Stainmesse, Serge, F-94600 Choisy Le Roi (FR); Fessi, Hatem, F-75007 Paris (FR); Devissaguet, Jean-Philippe, F-92200 Neuilly sur Seine (FR); Puisieux, Francis, F-94700 Maisons-Alfort (FR)
(74) Mandataire: Polus, Camille

(56) Documents cités:
- EP-A- 0 130 577
- EP-A- 0 190 050
- EP-A- 0 253 619
- EP-A- 0 274 961
- EP-A- 0 275 796
- WO-A-88/06438
- GB-A- 2 002 319
- STN FILE SUPPLIER (Karlsruhe) & FILE CA & CHEMICAL ABSTRACTS, vol. 102, 1984, no. 138256u, New York, US; L. RYDHAG et al.: "Colloidal stability of liposomes", & SURFACTANTS SOLUTION, [PROC. INT. SYMP.], 4TH. MEETING DATE 1982, VOLUME 3, 2039-57
- Biochim. Biophys. Acta, 266(1972). pp. 329-342
- Biochim. Biophys. Acta, 298 (1973), pp. 1015-1019
- Biochemistry, vol. 16, n 7 (1977), pp. 3932-3935
- Internat. Journal of Pharmaceuticals, vol. 95 (1993), pp. 51-56
- Liposome Technology, vol. I, preparation of liposomes. G. Gregoriadis (ed.), pp. 26, 29-35, 115, 116, 218, 251

## Description

La présente invention a pour objet un procédé de préparation de systèmes colloïdaux dispersibles de lipides amphiphiles, sous forme de liposomes oligolamellaires submicroniques.

On connaît de nombreux documents décrivant la préparation et l'utilisation des liposomes, notamment comme-véhicules de substances biologiquement actives telles que les médicaments, protéines, enzymes, agents de diagnostic ou produits cosmétiques. Ainsi, des substances hydrosolubles peuvent être encapsulées dans les espaces aqueux du liposome, ou des substances lipophiles peuvent être incorporées dans la paroi lipidique.

Un procédé de préparation de systèmes vésiculaires oligolamellaires a déjà été décrit par Bangham et col. (J. Mol. Biol. 13, 238-252; 1965). Selon ce procédé, les lipides et les substances lipophiles sont dissous dans un solvant organique et traités par une phase aqueuse sous forte agitation. Toutefois ce procédé, comme la plupart des procédés connus qui en dérivent, ne permet pas d'obtenir directement des liposomes de taille particulaire inférieure au micromètre, ce qui permettrait une bien plus grande stabilité des particules et de leurs dispersions.

EP-A-0 130 577 décrit un procédé de préparation de liposomes en mélangeant les composants membranaires avec un solvant non volatil et dispersant le mélange résultant dans un milieu aqueux à température elevée.

L'invention fournit un procédé simple et applicable à grande échelle, de préparation de liposomes de taille submicroscopique.

L'invention concerne donc un procédé de préparation de systèmes colloïdaux dispersibles de lipides amphiphiles, sous forme de liposomes oligolamellaire submicroniques , dont la paroi est constituée par lesdits lipides et éventuellement d'une substance A et dont le noyau est constitué parde de l'eau ou d'une solution aqueuse, et contenant éventuellement une substance B, caractérisé en ce que :
(1) on prépare une phase liquide constituée essentiellement par une solution des lipides amphiphiles et éventuellement de la substance A dans un solvant volatil ou dans un mélange de solvants volatils, et pouvant contenir la substance B en solution, la concentration des lipides dans le solvant volatil etant de 0,1 à 10 % en poids
(2) on prépare une seconde phase liquide constituée essentiellement par de l'eau ou une solution aqueuse de la substance B,
(3) on ajoute, sous agitation modérée, sans utilisation de seringue d'Hamilton la première phase à la seconde phase, de manière à obtenir, pratiquement instantanément, une suspension colloïdale de liposomes;
(4) si l'on désire, on élimine tout ou partie du solvant ou du mélange de solvants et de l'eau, de manière à obtenir une suspension colloïdale de concentration voulue en liposomes.

La substance A, de nature lipophile, est destinée à modifier les caractéristiques physiques (charge électrique, rigidité) ou chimique de la paroi. Elle peut être du cholestérol, de la stéarylamine, de l'acide phosphatidique, de l'alpha-tocophérol, un tensioactif non ionique, etc...

La substance B est une substance biologiquement active, notamment un principe actif médicamenteux ou un précureeur médicamenteux, un réactif biologique ou un produit cosmétique. La substance B est introduite dans la phase (1) si elle est lipophile et dans la phase (2) si elle est hydrophile.

Les lipides amphiphiles peuvent être des glycolipides, des phospho-aminolipides, et notamment les phospholipides, par exemple les lécithines (d'oeuf, de soja, etc...)..

Le solvant volatil est de préférence un alcool miscible à l'eau en toutes proportions, notamment l'éthanol.

La concentration des lipides dans le solvant peut être de préférence 1 à 5% en poids.

Il est avantageux que le volume de solvant utilisé pour la phase (1) soit compris entre 5 et 100%, par exemple environ 50%, du volume d'eau de la phase (2), afin d'obtenir des liposomes de petite taille (notamment de 100 à 300 nm).

On entend par "agitation modérée" une agitation telle que l'agitation magnétique, de 10 à 500 rpm, par exemple environ 100 rpm.

Ainsi l'invention permet d'obtenir des médicaments, notamment sous forme injectable, et des produits cosmétiques qui sont très stables.

Les exemples suivants illustrent l'invention.

### Exemple 1 : Préparation de liposomes.

| Phase organique 1 | |
|---|---|
| lécithine de soja (Epikuron 170) | 2,0 9 |
| éthanol absolu | 50,0 g |

| Phase aqueuse 2 | |
|---|---|
| Eau | 100,0 g |

La phase 1 est ajoutée sous agitation magnétique à la phase 2. Le milieu devient immédiatement opalescent parformation de liposomes. La taille moyenne des liposomes, mesurée immédiatement après préparation, dans un diffractomètre à rayon laser (Nanosizer^{R} de Coultronics), est de 180 nm, avec un indice moyen de dispersion de 0,5.

L'alcool est éliminé sous pression réduite, et la suspension de liposome est filtrée sur un verre fritté (pores 9-15 nm).

La taille des liposomes, à nouveau mesurée dans le filtrat demeure inchangée.

L'examen en microscopie à transmission montre des liposomes oligolamellaires de taille homogène.

### Exemple 2 : Préparation de liposomes contenant du cholestérol (variante de l'exemple 1).

On procède comme dans l'exemple 1, mais en ajoutant 0,30 g de cholestérol à la phase alcoolique. Les liposomes obtenus présentant les mêmes caractéristiques que dans l'exemple 1.

### Exemple 3 : Variante de l'exemple 1.

On procède comme dans l'exemple 1, mais en remplaçant la lécithine de soja par de la lécithine d'oeuf. Les liposomes obtenus présentent les mêmes caractéristiques que dans l'exemple 1.

### Exemple 4 : Variante de l'exemple 2.

On procède comme dans l'exemple 2, mais en remplaçant la lécithine de soja par de la lécithine d'oeuf. Les liposomes obtenus présentent les mêmes caractéristiques que dans l'exemple 1.

### Exemple 5 : Préparation de liposomes contenant un principe actif hydrophile.

On procède comme dans l'exemple 2, mais en ajoutant 0,20 g d'ampicilline (sel de sodium) dans la phase aqueuse.

Le taux d'incorporation de l'ampicilline dans les liposomes, mesurée après séparation des liposomes de la phase aqueuse par chromatographie sur gel Sephadex, est de 10%.

### Exemple 6 : Préparation de liposomes contenant un principe actif lipophile.

On procède comme dans l'exemple 1, mais en ajoutant 66,7 mg de muramyl-tripeptide-cholestérol à la phase organique. Le taux d'incorporation du principe actif est de 100%.

## Revendications

1. Procédé de préparation de systèmes colloïdaux dispersibles de lipides amphiphiles, sous forme de liposomes oligolamellaires submicroniques, dont la paroi est constituée par lesdits lipides et éventuellement d'une substance A et dont le noyau est constitué par de l'eau ou d'une solution aqueuse, et contenant éventuellement une substance B, caractérisé en ce que :
(1) on prépare une phase liquide constituée essentiellement par une solution desdits lipides et éventuellement de la substance A dans un solvant volatil choisi parmi les alcools miscibles à l'eau en toutes proportions ou dans un mélange de tels solvants volatils, et pouvant contenir la substance B en solution, la concentration des lipides dans le solvant volatil etant de 0,1 à 10 % en poids
(2) on prépare une seconde phase liquide constituée essentiellement par de l'eau ou une solution aqueuse de la substance B,
(3) on ajoute, sous agitation modérée, sans utilisation de seringue d'Hamilton, la première phase à la seconde phase, de manière à obtenir, pratiquement instantanément, une suspension colloïdale de liposomes.

2. Procédé selon la revendication 1, caractérisé en ce que les lipides amphiphiles sont des phospholipides.

3. Procédé selon l'une des revendications 1 ou 2, caractérisé en ce que l'alcool est l'éthanol.

4. Procédé selon la revendication 1, caractérisé en ce que la concentration des lipides dans le solvant est de 1 à 5 % en poids,

5. Procédé selon l'une quelconque des revendications 1 à 4, caractérisé en ce que le volume de solvant de la phase (1) est compris entre 5 et 100 % du volume aqueux de la phase (2).

6. Procédé selon l'une quelconque des revendications 1 à 5, caractérisé en ce que la substance A est le cholestérol.

7. Procédé selon l'une quelconque des revendications 1 à 5, caractérisé en ce que la substance B est un médicament hydrosoluble.

8. Procédé selon l'une quelconque des revendications 1 à 7, caractérisé en ce que les liposomes ont une taille d'environ 100 à 300 nm.

9. Procédé selon l'une quelconque des revendications 1 à 8, caractérisé en ce que, après l'étape (3), on élimine tout ou partie du solvant ou du mélange de solvants et de l'eau, de manière à obtenir une suspension colloïdale de concentration voulue en liposomes.

## Claims

1. Preparation process for dispersible colloidal systems of amphiphilic lipids, in the form of oligolamellar liposomes of submicron dimensions, the wall of which is constituted by the said lipids and optionally by a substance A and the nucleus of which is constituted by water or an aqueous solution, and optionally containing a substance B, characterized in that:
(1) a liquid phase is prepared constituted essentially by a solution of the said lipids and optionally by substance A in a volatile solvent chosen from alcohols miscible with water in any proportions or in a mixture of such volatile solvents, and which may contain substance B in solution, the concentration of the lipids in the volatile solvent being from 0.1 to 10% by weight,
(2) a second liquid phase is prepared constituted essentially by water or an aqueous solution of substance B,
(3) the first phase is added to the second phase, under moderate agitation, without the use of a Hamilton syringe, so as to obtain, almost immediately, a colloidal suspension of liposomes.

2. Process according to claim 1, characterized in that the amphiphilic lipids are phospholipids.

3. Process according to one of claims 1 or 2, characterized in that the alcohol is ethanol.

4. Process according to claim 1, characterized in that the concentration of lipids in the solvent is from 1 to 5% by weight.

5. Process according to any one of claims 1 to 4, characterized in that the volume of solvent of phase (1) is comprised between 5 and 100% of the aqueous volume of phase (2).

6. Process according to any one of claims 1 to 5, characterized in that substance A is cholesterol.

7. Process according to any one of claims 1 to 5, characterized in that substance B is a water-soluble medicament.

8. Process according to any one of claims 1 to 7, characterized in that the liposomes have a size of about 100 to 300 nm.

9. Process according to any one of claims 1 to 8, characterized in that, after stage (3), all or part of the solvent or of the mixture of solvents and water is eliminated, so as to obtain a colloidal suspension of liposomes of the desired concentration.

## Patentansprüche

1. Verfahren zur Herstellung von dispergierbaren kolloldalen Systemen aus amphiphilen Lipiden in Form von oligolamellaren Liposomen In Submikrongröße. deren Wand aus den genannten Lipiden und gegebenenfalls einer Substanz A gebildet ist und deren Kern aus Wasser oder einer wäßrigen Lösung gebildet ist und die gegebenenfalls eine Substanz B enthalten können, **dadurch gekennzeichnet**, daß man
(1) eine flüssige Phase herstellt, die im wesentlichen aus einer Lösung der genannten Lipide und gegebenenfalls der Substanz A in einem flüchtigen Lösungsmittel ausgewählt aus mit Wasser in sämtlichen Mengenverhältnissen mischbaren Alkoholen oder in einer Mischung solcher flüchtigen Lösungsmittel gebildet ist und die Substanz B in Lösung enthalten kann, wobei die Konzentration der Lipide in dem flüchtigen Lösungsmittel 0,1 bis 10 Gew.-% beträgt.
(2) eine zweite flüssige Phase herstellt, die im wesentlichen aus Wasser oder einer wäßrigen Lösung der Substanz B besteht.
(3) unter mäßigem Bewegen und ohne Verwendung einer Hamilton-Spritze die erste Phase in der Weise zu der zweiten Phase gibt, daß man praktisch augenblicklich eine kolloidale Suspension von Liposomen erhält.

2. Verfahren nach Anspruch 1, **dadurch gekennzeichnet**, daß die amphiphilen Lipide Phospholipide sind.

3. Verfahren nach einem der Ansprüche 1 oder 2, **dadurch gekennzeichnet**, daß der Alkohol Ethanol ist.

4. Verfahren nach Anspruch 1, **dadurch gekennzeichnet**, daß die Konzentration der Lipide in dem Lösungsmittel 1 bis 5 Gew.-% beträgt.

5. Verfahren nach irgendeinem der Ansprüche 1 bis 4, **dadurch gekennzeichnet**, daß das Volumen des Lösungsmittels der Phase (1) zwischen 5 und 100 % des wäßrigen Volumens der Phase (2) beträgt.

6. Verfahren nach irgendeinem der Ansprüche 1 bis 5, **dadurch gekennzeichnet**, daß die Substanz A Cholesterin ist.

7. Verfahren nach irgendeinem der Ansprüche 1 bis 5, **dadurch gekennzeichnet**, daß die Substanz B ein wasserlösliches Arzneimittel ist.

8. Verfahren nach irgendeinem der Ansprüche 1 bis 7, **dadurch gekennzeichnet**, daß die Liposomen eine Größe von etwa 100 bis 300 nm besitzen.

9. Verfahren nach irgendeinem der Ansprüche 1 bis 8, **dadurch gekennzeichnet**, daß man nach der Stufe (3) die Gesamtmenge oder einen Teil des Lösungsmittels oder der Lösungsmittelmischung und des Wassers entfernt, so daß man eine kolloidale Suspension mit der gewünschten Liposomen-Konzentration erhält.
